(19) Europäisches Patentamt
European Patent Office
Office européen des brevets

(11) Numéro de publication: **0 303 545 B1**

(12) **FASCICULE DE BREVET EUROPEEN**

(45) Date de publication de fascicule du brevet: **17.06.92**

(51) Int. Cl.[5]: **C07C 217/54**, C07C 229/34

(21) Numéro de dépôt: **88402094.2**

(22) Date de dépôt: **11.08.88**

(54) **Procédé pour la préparation de phényléthanolaminotétralines.**

(30) Priorité: **12.08.87 FR 8711497**
**14.06.88 FR 8807947**
**30.03.88 FR 8804219**

(43) Date de publication de la demande:
**15.02.89 Bulletin 89/07**

(45) Mention de la délivrance du brevet:
**17.06.92 Bulletin 92/25**

(84) Etats contractants désignés:
**AT BE CH DE ES FR GB GR IT LI LU NL SE**

(56) Documents cités:
**EP-A- 0 211 721**
**EP-A- 0 253 257**
**DE-A- 2 803 582**

(73) Titulaire: **ELF SANOFI**
**32-34, rue Marbeuf**
**F-75008 Paris(FR)**
(84) Etats contractants désignés:
**BE CH DE ES FR GB GR LI LU NL SE AT**

(73) Titulaire: **MIDY S.p.A.**
**Via Piranesi 38**
**I-20137 Milano(IT)**
(84) Etats contractants désignés:
**IT**

(72) Inventeur: **Boigegrain, Robert**
**Lou Pradas N 20 Jacou**
**F-34170 Castelnau le Lez(FR)**
Inventeur: **Cecchi, Roberto**
**Via Dei Tigli 1**
**I-20075 Lodi-Milan(IT)**
Inventeur: **Boveri, Sergio**
**Corso Repubblica, 48**
**I-15057 Tortona (Alessandria)(IT)**

(74) Mandataire: **Gillard, Marie-Louise et al**
**Cabinet Beau de Loménie 55, Rue d'Amster-dam**
**F-75008 Paris(FR)**

**EP 0 303 545 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne un procédé pour la préparation de phényléthanolaminotétralines, plus particulièrement par amidation d'un acide mandélique avec une aminotétraline et réduction, les intermédiaires utilisés dans ce procédé.

Le brevet européen 211721 décrit des phényléthanolaminotétralines de formule

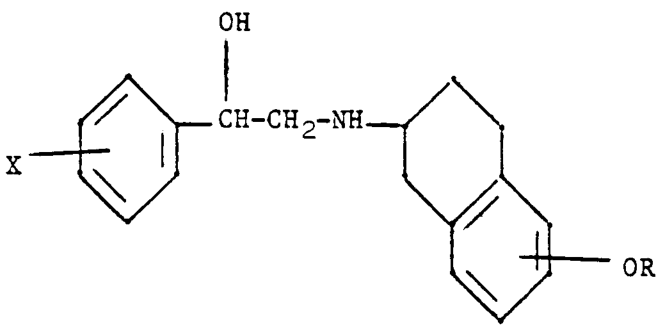

(A)

dans laquelle X représente l'hydrogène, un halogène, un groupe trifluorométhyle ou un groupe alkyle inférieur et R représente l'hydrogène; un groupe alkyle inférieur non substitué ou substitué par un groupe cycloalkyle contenant 3 à 7 atomes de carbone, un groupe hydroxy, alcoxy inférieur, carboxy ou carbalcoxy inférieur; un groupe cycloalkyle contenant 3 à 7 atomes de carbone; ou un alcanoyle inférieur et leur sels pharmaceutiquement acceptables.

Selon ce document, les composés de formule (A) et leurs sels pharmaceutiquement acceptables ont des propriétés pharmacologiques intéressantes, les composés ayant le substituant OR en position 7 de la tétraline ayant montré une activité lipolytique particulièrement prononcée.

Dans la présente description:

- le terme "alkyle inférieur", désigne un radical monovalent d'un hydrocarbure saturé contenant 1 à 4 atomes de carbone, tel que méthyle, éthyle, propyle, isopropyle ou n-butyle;
- le terme "carbalcoxy inférieur", désigne le groupe carboxyle estérifié avec un alkyle inférieur tel que défini ci-dessus;
- le terme "halogène" comprend les quatre halogènes fluor, chlore, brome, iode, les trois premiers étant particulièrement préférés;
- les termes "tétraline" et "tétralone" se rapportent au 1,2,3,4-tétrahydronaphtalène.

Selon le brevet européen ci-dessus, les produits de formule (A) sont préparés selon divers modes opératoires qui comportent toujours la réaction d'une 2-aminotétraline ou d'une 2-oxotétraline avec

- une phényléthanolamine ou
- un époxyde de styrène ou
- un phénylglyoxal ou
- une alpha-haloacétophénone.

Parmi les méthodes de préparation des produits de formule (A) ci-dessus, est décrite une O-alkylation qui, à partir du 2-[(7-hydroxy-1,2,3,4-tétrahydronapht-2-yl)amino]-1-phényléthanol ou du 2-[(7-hydroxy-1,2,3,4-tétrahydronapht-2-yl)amino]-1-(3-chlorophényl)éthanol, par réaction avec du bromoacétate d'éthyle donne le 2-[(7-carbéthoxyméthoxy-1,2,3,4-tétrahydronapht-2-yl)amino]-1-phényléthanol ou le 2-[(7-carbéthoxyméthoxy-1,2,3,4-tétrahydronapht-2-yl)amino]-1-(3-chlorophényl)éthanol. Cette réaction ne donne cependant pas de bons résultats car le rendement en produit final est très faible.

Toujours selon le brevet européen ci-dessus, les produits de formule (A) peuvent être préparés sous forme de diastéréoisomères ou de stéréoisomères optiquement purs en utilisant un ou les deux composés de départ sous forme optiquement active. Toutefois, l'obtention de stéréoisomères optiquement actifs comporte en général des cristallisations successives au niveau du produit final qui diminuent les rendements et, surtout, entrainent des opérations assez laborieuses.

On a maintenant trouvé qu'en faisant réagir une 2-amino-7-hydroxytétraline avec un acide mandélique on obtient un amide sous forme d'un mélange de racémates qui sont entre eux en rélation diastéréoisomérique et qui peuvent être facilement séparés.

On a également trouvé que les amides ainsi obtenus peuvent être réduits pour obtenir les phényléthanolaminotétralines sans qu'on interfère avec la stéréochimie des produits.

On a aussi trouvé qu'en partant d'une aminotétraline racémique et d'un acide mandélique optiquement

actif, ou vice-versa, on obtient un amide sous forme de diastéréoisomères facilement détectables et séparables par chromatographie qui, après séparation, donnent, par réduction, les phényléthanolaminotétralines optiquement pures.

On a enfin trouvé que l'amide préparé par réaction d'un acide mandélique avec une 2-amino-7-hydroxytétraline peut étre O-alkylé par un haloacétate d'alkyle inférieur avec de très bons rendements et que le produit ainsi obtenu peut être réduit pour préparer la O-carbalcoxyméthylphényléthanolaminotétraline correspondante avec un rendement global supérieur à celui de la O-alkylation décrite dans EP 211 721.

Ainsi, la présente invention concerne un procédé pour la préparation de phényléthanolaminotétralines de formule

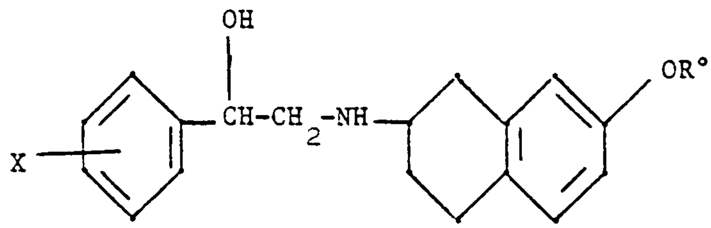

I

dans laquelle X est tel que défini ci-dessus et R° représente l'hydrogène ou un groupe méthyle substitué par un groupe carboxy ou carbalcoxy inférieur, et de leurs sels pharmaceutiquement acceptables, caractérisé en ce qu'on traite un dérivé fonctionnel d'un acide mandélique de formule

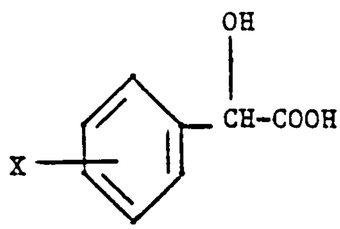

II

dans laquelle X est tel que défini ci-dessus, avec une aminotétraline de formule

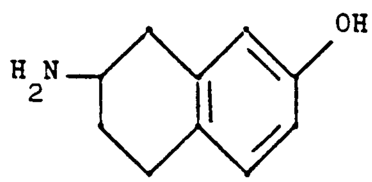

. III ;

le mandélamine ainsi obtenu de formule

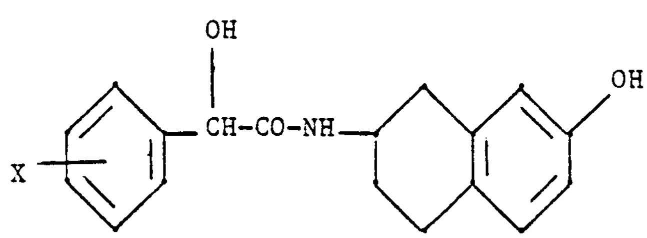

IV

dans laquelle X est tel que défini ci-dessus, est alors soumis à une réduction pour la transformation de son groupe amido en groupe méthylèneamino ou bien à une réaction, avec un haloacétate d'alkyle inférieur en présence d'un agent de condensation basique, ledit haloacétate étant un bromo-, chloro- ou iodoacétate ; dans cette seconde hypothèse, on soumet le produit obtenu, dans un ordre quelconque, à une réduction pour la transformation de son groupe amido en groupe méthylèneamino et à une saponification éventuelle

du groupe carbalcoxy inférieur en groupe carboxy ; et on transforme éventuellement le produit ainsi obtenu en l'un de ses sels pharmaceutiquement acceptables.

Parmi les composés qui peuvent être préparés selon le procédé de la présente invention, ceux de formule I dans laquelle X est tel que défini ci-dessus et R° est l'hydrogène ou un groupe carbéthoxyméthyle sont particulièrement préférés.

Comme dérivé fonctionnel de l'acide mandélique, on peut utiliser le chlorure, l'anhydride, un anhydride mixte, un ester actif ou l'acide libre opportunément activé, par exemple, avec le dicyclohexylcarbodiimide ou avec l'hexafluorophosphate de benzotriazolyl-N-oxytris-(diméthylamino)phosphonium (BOP). De préférence, on utilise comme composés de départ un acide mandélique activé avec un agent de condensation tel que le BOP.

La réaction du dérivé fonctionnel de l'acide mandélique avec la 2-amino-7-hydroxytétraline de formule III ci-dessus est conduite dans un solvant organique tel que le chlorure de méthylène, éventuellement en présence d'un accepteur de protons,tel que la triéthylamine.

De préférence, on utilise une quantité équimoléculaire d'acide mandélique, de BOP et d'aminotétraline.

On obtient ainsi le mandélamide correspondant de formule IV qui est isolé et, éventuellement, séparé en ses diastéréoisomères. Cette séparation est effectuée par chromatographie et chaque stéréoisomère ainsi séparé peut être utilisé pour l'étape suivante.

Ainsi, en partant d'un acide mandélique (II) et d'une 2-amino-7-hydroxytétraline (III) racémiques, on obtient un mélange de mandélamides diastéréoisomères qui, par séparation, donne le couple des diastéréoisomères RR,SS et RS,SR.

De même, en partant, par exemple, d'un acide mandélique optiquement actif et d'une aminotétraline (III) racémique, on obtient un mandélamide de formule IV sous forme de couple de diastéréoisomères (RR + RS) ou (SS + SR) qui, par séparation, donne les énantiomères purs (RR) et (RS) ou (SS) et (SR).

Comme produit de départ avantageux on utilise un acide (R)-mandélique, de préférence l'acide (R)-3-chloromandélique. On peut aussi utiliser l'acide 3-chloromandélique racémique.

Si l'acide mandélique de départ est sous forme racémique, l'aminotétraline de formule III sera de préférence utilisée sous forme optiquement active.

Les aminotétralines particulièrement préférées sont le (R)-2-amino-7-hydroxy-1,2,3,4-tétrahydronaphtalène et le (S)-2-amino-7-hydroxy-1,2,3,4-tétrahydronaphtalène.

On peut aussi utiliser le 2-amino-7-hydroxy-1,2,3,4-tétrahydronaphtalène racémique.

Le mandélamide de formule IV peut être directement soumis à la réduction pour la transformation du groupe amido en groupe méthylèneamino ou bien il est traité avec un haloacétate d'alkyle inférieur en milieu alcalin. La réaction de O-alkylation est conduite selon les modes opératoires connues en utilisant un chloroacétate, un bromoacétate ou un iodoacétate d'alkyle inférieur,le bromoacétate étant préféré. Comme agent de condensation alcalin, on peut utiliser un hydroxyde ou un carbonate d'un métal alcalin, par exemple le carbonate de potassium et on peut opérer en présence d'un catalyseur tel que l'iodure de potassium. L'éther (carbalcoxy inférieur)méthylique de l'amide IV est isolé selon les techniques conventionnelles avec de très bons rendements.

Le produit ainsi obtenu peut être directement soumis à la réduction du groupe amido en groupe méthylèneamino, ou bien il peut être saponifié pour transformer le groupe carbalcoxy inférieur en groupe carboxy, libre ou salifié, selon les techniques bien connues.

Ainsi, la réduction du groupe amido en groupe méthylèneamino peut être conduite sur un mandélamide de formule

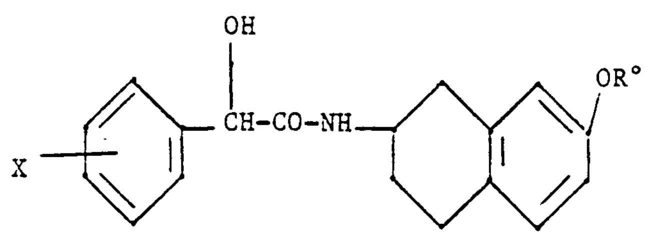

(IVa)

dans laquelle X et R° sont tels que définis ci-dessus.

L'étape de réduction du mandélamide de formule IVa est effectuée, par exemple, par action d'un hydrure tel que l'hydrure de lithium et d'aluminium ou du diborane, notamment d'un réactif générant le diborane tel que le complexe entre le borane et le diméthylsulfure, ci-après désigné "borane-méthylsulfure". La réaction est conduite dans un solvant organique, tel que le tétrahydrofuranne et le composé de

formule I est isolé selon les techniques connues.

Dans le cas de la réduction d'un mandélamide de formule IVa dans laquelle R° représente un méthyle substitué par un groupe carboxy éventuellement salifié ou, notamment ,un carbalcoxy inférieur, ce groupe peut également être réduit en l'alcool. Il est donc souhaitable, dans ce cas, d'utiliser un réactif de réduction qui permet d'obtenir, au moins de préférence, la réduction sélective du groupe amide. De toute façon on obtient, surtout lorsqu'on réduit un mandélamide de formule IVa dans laquelle R° est un méthyle substitué par un groupe carbalcoxy, un mélange d'une part du produit de formule I dans laquelle R° est un méthyle substitué par le groupe carbalcoxy inférieur correspondant et, d'autre part, du produit de formule

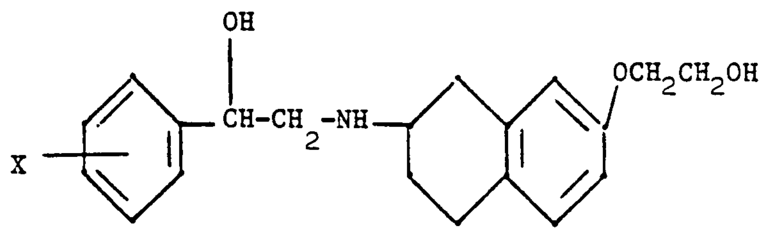

(Ia)

dans laquelle X est tel que défini ci-dessus.

Les produits de formule Ia sont nouveaux et possèdent une bonne activité sur la motricité intestinale.

Si, comme réactif de réduction, on utilise le borane-méthylsulfure et si l'on opère à basse température (15-25°C) on obtient de préférence la réduction du groupe amido et le composé correspondant de formule I, où R° est un groupe méthyle substitué par un groupe carboxy ou carbalcoxy inférieur, est obtenu avec des rendements globaux supérieurs à ceux décrits dans EP 211 721.

La réaction de réduction respecte la stéréochimie du mandélamide IVa et les produits de formule I peuvent être ainsi obtenus sous forme de mélange racémique ou sous forme de diastéréoisomères ou d'isomères optiquement purs à partir du mandélamide approprié.

La 2-amino-7-hydroxytétraline de formule III est préparée à partir de la méthoxytétralone correspondante de formule

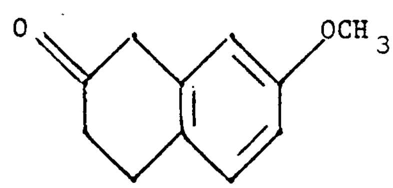

V

par réaction avec la benzylamine, réduction par du borohydrure de sodium de la benzylimine ainsi obtenue, débenzylation par hydrogénation catalytique et déméthylation par l'acide bromhydrique à 48%.

Les deux formes optiquement actives des aminotétralines de formule III sont préparées par résolution des racémates selon des méthodes connues, par exemple par salification avec un acide optiquement actif, l'acide mandélique de préférence.

Les mandélamides de formule IVa ci-dessus sont nouveaux et représentent les intermédiaires clé du procédé de la présente invention.

Ainsi, la présente invention a pour objet, selon un autre de ses aspects, les composés de formule IVa, sous forme racémique ou sous forme de leurs stéréoisomères séparés.

Les exemples suivants illustrent l'invention sans toutefois la limiter. Le symbole du pouvoir rotatoire est indiqué comme [alpha], mais on doit l'entendre comme $[alpha]_D^{20}$ .

PREPARATION I

Bromhydrate de 2-amino-7-hydroxytétraline; SR 58518 A.

(a) On chauffe au reflux pendant 3 heures un mélange de 8 g de 7-méthoxy-2-tétralone, 4,8 g de benzylamine, 150 ml de toluène anhydre et 100 mg d'acide p.toluènesulfonique. On évapore à sec l'huile résiduelle, on reprend par 100 ml de méthanol et à la solution obtenue on ajoute, avec précaution et à 0-5°C, 8,5 g de borohydrure de sodium. On laisse le mélange sous agitation et à la température

ambiante pendant une nuit, puis on y ajoute 50 ml d'eau, on laisse sous agitation pendant 30 minutes, on évapore le solvant, on reprend avec 30 ml d'eau et 10 ml d'une solution concentrée d'hydroxyde d'ammonium. On extrait avec 200 ml d'acétate d'éthyle, on sèche la phase organique sur du sulfate de sodium, on filtre et évapore à sec. On obtient une huile de couleur foncée qui est purifiée par flash chromatographie en utilisant comme éluant le mélange acétate d'éthyle/méthanol 95/5. On transforme la base obtenue en son chlorhydrate par dissolution dans 40 ml d'isopropanol et addition d'isopropanol saturé d'acide chlorhydrique gazeux. On obtient ainsi 11,4 g de chlorhydrate de 7-méthoxy-2-benzylamino-1,2,3,4-tétrahydronaphtalène; p.f. 265-267 ° C (déc.).

(b) On soumet le produit ci-dessus, dissous dans 200 ml de méthanol et 100 ml d'eau, à une hydrogénation en présence de 1,2 g de palladium sur charbon à 10%, à la pression ambiante et à la température de 45-50 ° C. Après 4 heures on filtre, évapore à sec, reprend deux fois avec de l'éthanol absolu et l'on évapore à sec. On obtient un solide blanc qui est repris avec 70 ml d'isopropanol à chaud. Par refroidissement, la suspension obtenue précipite et donne 7,8 g de chlorhydrate de 2-amino-7-méthoxytétraline; p.f. 214-216 ° C.

(c) On met en suspension 6,6 g du produit ci-dessus dans 80 ml d'acide bromhydrique à 48% et on chauffe le mélange au reflux pendant 2 heures. On évapore à sec la solution obtenue, on reprend le résidu par de l'éthanol absolu et on évapore à sec deux fois. On obtient ainsi une huile qu'on dissout dans 20 ml d'isopropanol à chaud. Par addition de 30 ml d'éther éthylique à la solution on obtient 6,8 g de bromhydrate de 2-amino-7-hydroxytétraline cristallin; p.f. 171-173 ° C.

PREPARATION II

Monohydrate de R( + )-2-amino-7-hydroxytétraline; SR 58554.

A une solution dans 550 ml d'éthanol absolu de 50 g de 2-amino-7-méthoxytétraline base brute, obtenue du chlorhydrate correspondant (PREPARATION I b) par neutralisation avec de l'hydroxyde de sodium à 10%, extraction avec de l'acétate d'éthyle et évaporation du solvant, on ajoute une solution de 43 g d'acide ( + ) mandélique dans 550 ml d'éthanol absolu. Après une nuit à la température ambiante, on filtre le précipité obtenu et on le cristallise deux fois dans l'éthanol absolu en recupérant chaque fois le produit cristallisé après repos d'une nuit à la température ambiante. On obtient ainsi 34,2 g (74%) de sel pur de l'acide ( + ) mandélique avec la ( + )-2-amino-7-méthoxytétraline; p.f. 190-192 ° C. Les eaux mères de cette première cristallisation sont séparées et utilisées pour la PREPARATION III cidessous. On suspend 34 g du sel ainsi obtenu dans 300 ml d'eau et on rend basique le mélange réactionnel avec de l'hydroxyde de sodium N. On extrait la base avec de l'acétate d'éthyle, on évapore à sec et on reprend le residu par 260 ml d'acide bromhydrique à 48%. On chauffe au reflux le mélange réactionnel pendant trois heures, on l'évapore à sec sous pression réduite et on reprend le résidu obtenu avec 70 ml d'eau. On rend basique la solution aqueuse avec de l'hydroxyde d'ammonium con- centré, on la refroidit pendant une nuit et on la filtre. On obtient 17 g de R( + )-2-amino-7-hydroxytétraline sous forme monohydratée, SR 58554; p.f. 143-144 ° C, [alpha] = + 85,1 ° (méthanol, c = 0,5 %).

Le chlorhydrate de ce produit a un pouvoir rotatoire qui correspond à celui de la littérature (Molecular Pharmacology, 1982, 22, 281-289).

PREPARATION III

Monohydrate de s(-)-2-amino-7-hydroxytétraline, SR 58555.

On évapore à sec les eaux mères de la première cristallisation du produit SR 58554 (PREPARATION II), on suspend le résidu ainsi obtenu dans 300 ml d'eau et on rend le mélange basique par de l'hydroxyde de sodium N. On extrait la base avec de l'acétate d'éthyle. Suivant le mode opératoire décrit dans la PREPARATION II et en utilisant comme produits de départ la base ainsi obtenue et l'acide (-) mandélique, on obtient le sel de l'acide (-) mandélique avec la (-)-2-amino-7-méthorytétraline (p.f. 189-191 ° C) qui, par traitement avec l'hydroxyde de sodium N et déméthylation avec de l' acide bromhydrique, donne 17 g de S(-)-2-amino-7-hydrorvtétraline sous forme de monohydrate, SR 58555; p.f. 143-144 ° C, [alpha] = - 86,9 ° (méthanol, c = 0,5 %).

Le chlorhydrate de ce produit a un pouvoir rotatoire qui correspond à celui de la littérature (Molecular Pharmacology 1982, 22, 281-289).

EXEMPLE 1

(a) N-(7-hydroxy-1,2,3,4-tétrahydronapht-2-yl)-3-chloromandélamide,SR 58534 (mélange de diastéréoisomères)

A une suspension de 6,5 g de bromhydrate de 2-amino-7-hydroxytétraline (PREPARATION Ic), 4,98 g d'acide 3-chloromandélique et de 10,69 g de hexafluorophosphate de benzotriazolyl-N-oxytris-(diméthylamino)phosphonium (BOP) dans 120 ml de chlorure de méthylène, on ajoute lentement 7,44 ml de triéthylamine et on laisse réagir à la température ambiante sous agitation. Après trois heures, on ajoute 100 ml d'une solution saturée de chlorure de sodium et on laisse le mélange sous agitation pendant 30 minutes à la température ambiante. On ajoute 200 ml d'acétate d'éthyle et on sépare les deux phases. On lave la phase organique avec une solution d'acide chlorhydrique 2N, puis avec une solution saturée de bicarbonate de sodium et ensuite avec une solution saturée de chlorure de sodium. On sèche la phase organique sur du sulfate de sodium, on la concentre sous pression réduite et on obtient 12 g d'une huile marron impure détectée par chromatographie couche mince (CCM). On purifie l' huile par flash chromatographie en utilisant comme éluant un mélange acétate d'éthyle/cyclohexane 6/4 et on réunit toutes les fractions. On récupère 7,5 g (84%) d'une huile jaune pâle, pure en CCM, qu'on solidifie à la pompe et égrène dans l'éther.

(b) (RS,SR)-N-(7-hydroxy-1,2,3,4-tétrahydronapht-2-yl)-3-chloromandélamide, SR 58535 (diastéreoisomère moins polaire).

On dissout à chaud 6 g du mélange diastéréoisomérique SR 58534 obtenu comme décrit ci-dessus dans 80 ml d'éther éthylique. Après une nuit dans le réfrigérateur, on obtient un précipité qu'on filtre. On obtient 1,4 g d'un solide blanc qui, en CCM (éluant acétate d'éthyle/ cyclohexane 1/1), correspond à la tache moins polaire des deux taches du mélange SR 58534. On soumet les eaux mères à la flash chromatographie en éluant avec un mélange acétate d'éthyle/ cyclohexane 1/1. On obtient 1,2 g d'un produit solide correspondant à la tache du produit moins polaire, 1,8 g de mélange et 1,9 g du produit correspondant à la tache du produit plus polaire. On cristallise 2,6 g de produit correspondant à l'isomère moins polaire dans 60 ml d'acétate d'éthyle et l'on obtient 2,1 g du premier isomère pur; p.f. 172-174 ° C.

(c) (RR,SS)-N-(7-hydroxy-1,2,3,4-tétrahydronapht-2-yl)-3-chloromandélamide, SR 58536 (diastéreoisomère plus polaire).

L'isomère plus polaire obtenu comme décrit dans (b), SR 58536, est une huile blanche qui, en CCM, présente le même déplacement que l'isomère (RR), SR 58533, décrit dans l'Exemple 20 ci-dessous. Les 1,9 g de l'huile ainsi obtenue, dissous dans un mélange de 15 ml d'acétate d'éthyle et de 5 ml d'éther éthylique, laissés 48 heures à la température ambiante, donnent le SR 58536 cristallin; p.f. 136-138 ° C.

## EXEMPLE 2

N-(7-hydroxy-1,2,3,4-tétrahydronapht-2-yl)-4-chloromandélamide.

A un mélange de 3,2 g de bromhydrate de 2-amino-7-hydroxytétraline (PREPARATION Ic), 2,4 g d'acide 4-chloromandélique et 5,2 g de hexafluorophosphate de benzotriazolyl-N-oxytris-(diméthylamino) phosphonium dans 50 ml de chlorure de méthylène, on ajoute 3,6 ml de triéthylamine. On laisse la solution obtenue sous agitation à la température ambiante pendant 3 heures, on ajoute une solution saturée de chlorure de sodium et on agite pendant 30 minutes. On évapore à sec la phase organique qui se sépare et on obtient une huile qu'on dissout dans 200 ml d'acétate d'éthyle. On lave la solution obtenue deux fois avec 40 ml d'une solution d'acide chlorhydrique 2N, deux fois avec une solution saturée de bicarbonate de sodium et, ensuite, avec une solution saturée de chlorure de sodium. On sèche la solution sur du sulfate de sodium, on la filtre, on l'évapore à sec et on chromatographie l'huile obtenue en utilisant comme éluant un mélange acétate d'éthyle/cyclohexane 1/1 et on réunit toutes les fractions. On obtient 2,7 g (63%) de N-(7-hydroxy-1,2,3,4-tétrahydronapht-2-yl)-4-chloromandélamide, comme mélange diastéréoisomèrique; p.f. 170 ° C environ (large interval de fusion), suffisamment pur pour la réaction ultérieure. Dans ce mélange, les diastéreoisomères (RS,SR) et (RR,SS) sont bien visibles en chromatographie; ils peuvent être facilement séparés et obtenus à l'état pur.

## EXEMPLE 3

(a) Mélange des stéreoisomères
N-[(2R)-7-hydroxy-1,2,3,4-tétrahydronapht-2-yl]-(R)-mandélamideet
N-[(2S)-7-hydroxy-1,2,3,4-tétrahydronapht-2-yl]-(R)-mandélamide, SR 58542.

A une solution de 10 g de bromhydrate de 2-amino-7-hydroxytétraline (PREPARATION Ic), 6,3 g d'acide

(R)-mandélique et 16 g de BOP dans 200 ml de chlorure de méthylène anhydre, on ajoute lentement 8,1 g de triéthylamine et on laisse la solution ainsi obtenue sous agitation à la température ambiante pendant 3 heures. On ajoute 100 ml d'une solution saturée de chlorure de sodium, on agite à la température ambiante pendant 30 minutes, on ajoute 400 ml d'acétate d'éthyle, on sépare les phases et on élimine la phase aqueuse. On lave la phase organique deux fois avec 50 ml d'acide chlorhydrique 2N, deux fois avec une solution saturée de bicarbonate de sodium et, ensuite, on la sèche sur du sulfate de sodium, on la filtre et on l'évapore à sec. On purifie l' huile obtenue par flash chromatographie en utilisant comme éluant un mélange acétate d'éthyle/cyclohexane 6/4 et on réunit toutes les fractions. On obtient 10,5 g (rendement 88%) du mandélamide SR 58542 sous forme d'une huile épaisse qui, en CCM, se revèle constituée par les deux diastéréoisomères dont le pourcentage n'a pas été déterminé.

(b) N-[(2R ou 2S)-7-hydroxy-1,2,3,4-tétrahydronapht-2-yl]-(R)-mandélamide, SR 58543, isomère moins polaire.

On subdivise 10,5 g du mélange de diastéréoisomères SR 58542, obtenu comme décrit ci-dessus, en trois portions de 3 g environ. On soumet chaque portion à la flash chromatographie en éluant avec un mélange acétate d'éthyle/cyclohexane 1/1. Les fractions obtenues des trois chromatographies, réunies, donnent 3,1 g d'une huile correspondant à la tache moins polaire, 3,2 g d'un solide correspondant à la tache plus polaire et 2 g d'un mélange des deux produits. On traite les 3,1 g de l'isomère moins polaire à la pompe mécanique et on obtient un solide vitreux ayant un point de fusion indéfini; [alpha] = -107° (méthanol, c = 0,5 %). Rendement de la séparation du mélange: 30%. Après 24 heures à la température ambiante, le solide vitreux cristallise; on le reprend avec de l'éther éthylique et le filtre; p.f. 157-159° C, sans modification de [alpha].

(c) N-[(2S ou 2R)-7-hydroxy-1,2,3,4-tétrahydronapht-2-yl]-(R)-mandélamide, SR 58544, isomère plus polaire.

On cristallise dans 20 ml d'acétate d'éthyle les 3,2 g du solide blanc correspondant à l'isomère plus polaire obtenu comme décrit dans (b). On obtient 2,8 g d'un produit ayant comme point de fusion 145-147° C; [alpha] = + 26° (méthanol, c = 1%).

EXEMPLE 4

N-(7-hydroxy-1,2,3,4-tétrahydronapht-2-yl)-2-(3-chlorophényl)-2-hydroxyéthanamine, SR 58339, mélange de diastéréoisomères.

A une suspension de 0,9 g d'hydrure de lithium et d'aluminium dans 20 ml de tétrahydrofuranne anhydre on ajoute lentement une solution de 1,8 g du N-(7-hydroxy-1,2,3,4-tétrahydronapht-2-yl)-3-chloromandélamide décrit dans l'Exemple 1 (a) (SR 58534), dans 30 ml de tétrahydrofuranne, et on laisse au reflux pendant 4 heures. On refroidit par un bain d'eau glacée, on ajoute lentement 2 ml d'eau, 2 ml d'une solution concentrée d'hydroxyde de sodium et 10 ml d'eau; on agite à la température ambiante pendant 20 minutes, on ajoute 50 ml d'acétate d'éthyle, on filtre sur CELITE (Marque enrégistrée) pour casser l'émulsion. On sèche sur du sulfate de sodium la phase organique qui se sépare, on filtre et évapore à sec. On obtient 1,2 g d'une huile qui est purifiée par flash chromatographie en utilisant comme éluant un mélange chlorure de méthylène/méthanol 9/1. On dissout la base purifiée dans 10 ml d'acétate d'éthyle et, par refroidissement, on obtient 0,35 g de SR 58339 base; p.f. 133-138° C; pourcentage des diastéréoisomères non défini. Dans ce mélange, les diastéréoisomères (RS,SR) et (RR,SS) ne sont pas visibles en chromatographie et ne peuvent être séparés que par d'autres techniques.

Son chlorhydrate, SR 58339A, est décrit dans l'Exemple 8 du brevet européen 211721.

EXEMPLE 5

(RS,SR)-N-(7-hydroxy-1,2,3,4-tétrahydronapht-2-yl)-2-(3-chlorophényl)-2-hydroxyéthanamine, SR 58523.

A une solution de 6 g de SR 58535, obtenu comme décrit dans l'Exemple 1 (b), dans 60 ml de tétrahydrofuranne anhydre, chauffée au reflux sous courant d'azote, on ajoute en 20 minutes 4,5 ml d'une solution 10 M de complexe borane-méthylsulfure, diluée dans 20 ml de tétrahydrofuranne anhydre, puis on ajoute goutte à goutte 40 ml de méthanol. On chauffe au reflux encore 30 minutes, puis on évapore à sec, on reprend avec 30 ml d'eau, 5 ml d'hydroxyde d'ammonium concentré et 200 ml d'acétate d'éthyle. On sépare la phase organique, on la sèche sur du sulfate de sodium et on l'évapore à sec. On soumet le résidu à une flash chromatographie en éluant avec un mélange chlorure de méthylène/méthanol 9/1. Par cristallisation du résidu solide dans 50 ml d'acétate d'éthyle, on obtient 1,2 g de produit fondant à 180-184°C qui, après recristallisation dans 60 ml d'acétate d'éthyle donne le SR 58523 pur; p.f. 184-186°C.

EXEMPLE 6

(RR,SS)-2-[(7-hydroxy-1,2,3,4-tétrahydronapht-2-yl)amino]-2-(3-chlorophényl)-2-hydroxyéthanamine, SR 58524.

Suivant le mode opératoire décrit dans l'Exemple 5 et en partant de 4 g de SR 58536, obtenu comme décrit dans l'Exemple 1(c), on obtient 2,4 g de SR 58524 pur, cristallisé dans l'isopropanol; p.f. 143-145°C

EXEMPLE 7

N-(7-hydroxy-1,2,3,4-tétrahydronapht-2-yl)-2-(4-chlorophényl)-2-hydroxyéthanamine, SR 58521, mélange de diastéréoisomères.

A une suspension de 0,9 g de hydrure de lithium et d'aluminium dans 20 ml de tétrahydrofuranne anhydre on ajoute, pendant 10 minutes sous courant d'azote, une solution de 2,2 g du mélange diastéréoisomérique de N-(7-hydroxy-1,2,3,4-tétrahydronapht-2-yl)-4-chloromandélamide obtenu comme décrit dans l'Exemple 2, dans 30 ml de tétrahydrofuranne anhydre. On chauffe au reflux pendant 3 heures, on ajoute 0,45 g d'hydrure de lithium et d'aluminium et on chauffe au reflux pendant 90 minutes. On refroidit à 5°C, on ajoute 50 ml d'éther éthylique, puis, goutte à goutte, 20 ml d'eau. On extrait avec 100 ml d'acétate d'éthyle, on sépare la phase organique, on la sèche sur du sulfate de sodium, on filtre et on évapore à sec. On obtient une huile qui est purifiée par flash chromatographie en utilisant comme éluant un mélange chlorure de méthylène/méthanol 85/15. On obtient un produit solide qu'on cristallise dans 20 ml d'acétate d'éthyle. On obtient 0,7 g du produit susdit comme mélange diastéréoisomèrique; p.f. 152-156°C (rendement 33%). Dans ce mélange, les diastéréoisomères (RS,SR) et (RR,SS) ne sont pas visibles en chromatographie et ne peuvent être séparés que par d'autres techniques.

EXEMPLE 8

(a) A une solution de 7 g de monohydrate de R(+)-2-amino-7-hydroxy-1,2,3,4-tétrahydronaphtalène (PREPARATION II), 7,2 g d'acide 3-chloromandélique racémique, 16 g de BOP dans 200 ml de chlorure de méthylène anhydre, on ajoute lentement 3,9 g de triéthylamine et on laisse la solution ainsi obtenue sous agitation à la température ambiante pendant 3 heures. On ajoute 100 ml d'une solution saturée de chlorure de sodium, on agite pendant 30 minutes à la température ambiante, on ajoute 400 ml d'acétate d'éthyle, on sépare les phases et on élimine la phase aqueuse. On lave la phase organique deux fois avec 50 ml d'acide chlorhydrique 2N, puis deux fois avec une solution saturée de bicarbonate de sodium et, ensuite, on la sèche sur du sulfate de sodium, on la filtre et on l'évapore à sec. On purifie le produit obtenu par flash chromatographie en utilisant comme éluant un mélange acétate d'éthyle/cyclohexane 6/4 et on réunit toutes les fractions. On obtient le mélange de diastéréoisomères N-[(2R)-7-hydroxy-1,2,3,4-tétrahydronapht-2-yl]-(RS)-3-chloromandélamide.

(b) On soumet 15 g du mélange de diastéréoisomères, obtenu comme décrit ci-dessus, à une flash chromatographie en éluant avec un mélange acétate d'éthyle/cyclohexane 1/1. On obtient 4,5 g d'un produit correspondant à la tache moins polaire, 5 g d'un produit correspondant à la tache plus polaire et 3 g d'un mélange des deux produits. On traite les 4,5 g de l'isomère moins polaire à la pompe mécanique et on obtient le N-[(2R)-7-hydroxy-1,2,3,4-tétrahydronapht-2-yl]-(S)-3-chloromandélamide, SR 58588, sous forme d'un solide ayant un point de fusion indéfini; [alpha] = +92,5° (méthanol, c = 1%).

(c) On cristallise dans 20 ml d'acétate d'éthyle les 5 g du solide blanc correspondant à l'isomère plus polaire obtenu comme décrit dans (b). On obtient 4,3 g de N-[(2R)-7-hydroxy-1,2,3,4-tétrahydronapht-2-yl]-(R)-3-chloromandélamide, SR 58533; p.f. 143-147°C; [alpha] = +35,1° (méthanol, c = 1%).

EXEMPLE 9

Suivant le mode opératoire décrit dans l'Exemple 8 et en remplacant le monohydrate de R(+)-2-amino-7-hydroxy-1,2,3,4-tétrahydronaphtalènepar le monohydrate de S(-)-2-amino-7-hydroxy-1,2,3,4-tétrahydronaphtalène (PREPARATION III), on obtient:

(a) N-[(2S)-7-hydroxy-1,2,3,4-tétrahydronapht-2-yl]-(Rs)-3-chloromandélamide, mélange de diastéréoisomères;

(b) N-[(2S)-7-hydroxy-1,2,3,4-tétrahydronapht-2-yl]-(R)-3-chloromandélamide, SR 58587; isomère moins polaire; point de fusion indéfini; [alpha] = -95° (méthanol, c = 1%);
(c) N-[(2S)-7-hydroxy-1,2,3,4-tétrahydronapht-2-yl]-(S)-3-chloromandélamide, SR 58574; p.f. 145-146°C; isomère plus polaire; [alpha] = -34,2° (méthanol, c = 1%);

EXEMPLE 10

(a) A une solution de 7,0 g de monohydrate de R(+)-2-amino-7-hydroxy-1,2,3,4-tétrahydronaphtalène (PREPARATION II), 5,8 g d'acide mandélique racémique, 15,3 g de BOP dans 200 ml de chlorure de méthylène anhydre, on ajoute lentement 3,9 g de triéthylamine et on laisse la solution ainsi obtenue sous agitation à la température ambiante pendant 3 heures. On ajoute 100 ml d'une solution saturée de chlorure de sodium, on agite pendant 30 minutes à la température ambiante, on ajoute 400 ml d'acétate d'éthyle, on sépare les phases et on élimine la phase aqueuse. On lave la phase organique deux fois avec 50 ml d'acide chlorhydrique 2N, deux fois avec une solution saturée de bicarbonate de sodium et, ensuite, on la sèche sur du sulfate de sodium, on la filtre et on l'évapore à sec. On purifie le produit obtenu par flash chromatographie en utilisant comme éluant le mélange acétate d'éthyle/cyclohexane 6/4 et on réunit toutes les fractions. On obtient le mélange de diastéréoisomères N-[(2R)-7-hydroxy-1,2,3,4-tétrahydronapht-2-yl]-(Rs)-mandélamide.
(b) On soumet 12 g du mélange de diastéréoisomères, obtenu comme décrit ci-dessus, à une flash chromatographie en éluant avec un mélange acétate d'éthyle/cyclohexane 1/1. On obtient 4 g d'un produit correspondant à la tache moins polaire, 4,5 g d'un produit correspondant à la tache plus polaire et 2 g d'un mélange des deux produits. On traite les 4 g de l'isomère moins polaire à la pompe mécanique et on obtient le N-[(2R)-7-hydroxy-1,2,3,4-tétrahydronapht-2-yl]-(S ou R)-mandélamide SR 58561; p.f. 159-160°C; [alpha] = +110° (méthanol, c = 0,5 %).
(c) On cristallise dans 20 ml d'acétate d'éthyle les 4,5 g du solide blanc correspondant à l'isomère plus polaire obtenu comme décrit dans (b). On obtient 3,9 g de N-[(2R)-7-hydroxy-1,2,3,4-tétrahydronapht-2-yl]-(R ou S)-mandélamide; p.f. 145-147°C; [alpha] = +26° (méthanol, c = 1%), identique au produit SR 58544 de l'Exemple 3 (c).

EXEMPLE 11

Suivant le mode opératoire décrit dans l'Exemple 10 et en remplaçant le mcnohydrate de R(+)-2-amino-7-hydroxy-1,2,3,4-tétrahydronaphtalènepar le monohydrate de S(-)-2-amino-7-hydroxy-1,2,3,4-tétrahydronaphtalène (PREPARATION III), on obtient:
(a) N-[(2S)-7-hydroxy-1,2,3,4-tétrahydronapht-2-yl]-(RS)-mandélamide; mélange de diastéréoisomères;
(b) N-[(2S)-7-hydroxy-1,2,3,4-tétrahydronapht-2-yl]-(R ou S)-mandélamide; p.f. 157-159°C; [alpha] = -107° (méthanol, c = 0,5 %), identique au produit SR 58543 de l'Exemple 3(b);
(c) N-[(2S)-7-hydroxy-1,2,3,4-tétrahydronapht-2-yl]-(S ou R)-mandélamide, SR 58559; p.f. 147-148°C; [alpha] = -27,5° (méthanol, c = 0,5 %).

EXEMPLE 12

A une solution de 3 g de N-[(2R)-7-hydroxy-1,2,3,4-tétrahydronapht-2-yl]-(S)-3-chloromandélamide, SR 58588, décrit dans l'Exemple 8(b), dans 40 ml de tétrahydrofuranne anhydre, chauffée au reflux sous courant d'azote, on ajoute 2,7 ml d'une solution 10M de borane-méthylsulfure dans 20 ml de tétrahydrofuranne, et on continue le chauffage au reflux pendant 4 heures. A la solution refroidie à la température ambiante, on ajoute 25 ml de méthanol et on laisse sous agitation d'abord 30 minutes à la température ambiante et, ensuite, 30 minutes au reflux. On concentre sous pression réduite et, après filtration, on cristallise deux fois dans l'isopropanol. On obtient 2,1 g (73%) de N-[(2R)-7-hydroxy-1,2,3,4-tétrahydronapht-2-yl]-(2S)-2-(3-chlorophényl)-2-hydroxyéthanamine, SR 58590; p.f. 186-188°C; [alpha] = +76° (méthanol, c = 0,5 %).

EXEMPLE 13

Suivant le mode opératoire décrit dans l'Exemple 12, en partant de 4,7 g de SR 58533, obtenu comme décrit dans l'Exemple 8(c), on obtient une base sous forme d'une huile résiduelle qu'on purifie par flash chromatographie en éluant avec un mélange chlorure de méthylène/méthanol 95/5. On dissout la base purifiée dans l'acétone, on filtre la solution et on ajoute de l'isopropanol saturé d'acide chlorhydrique. Après

filtration, on cristallise deux fois dans l'isopropanol. On refroidit le produit, on le filtre et on le lave d'abord avec de l'isopropanol et après avec de l'acétone. On obtient 3,2 g (65%) de chlorhydrate de la N-[(2R)-7-hydroxy-1,2,3,4-tétrahydronapht-2-yl]-(2R)-2-(3-chlorophényl)-2-hydroxyéthanamine, SR 58572 A; p.f. 203-205°C; [alpha] = +36,4° (méthanol, c = 1%).

EXEMPLE 14

Suivant le mode opératoire décrit dans l'Exemple 12, en partant de 2,5 g de SR 58587, obtenu comme décrit dans l'Exemple 9(b), on obtient 1,6 g (67%) de N-[(2S)-7-hydroxy-1,2,3,4-tétrahydronapht2-yl]-(2R)-2-(3-chlorophényl)-2-hydroxyéthanamine, SR 58589; p.f. 185-187°C; [alpha] = -78,5° (méthanol, c = 0,5 %).

EXEMPLE 15

Suivant le mode opératoire décrit dans l'Exemple 12 et en utilisant comme produit de départ 3,44 g de SR 58574, obtenu comme décrit dans l'Exemple 9(c), on obtient une base sous forme d'une huile résiduelle qu'on purifie par flash chromatographie en éluant avec un mélange chlorure de méthylène/méthanol 95/5. On dissout la base purifiée dans l'acétone, on filtre la solution et on ajoute de l'isopropanol saturé d'acide chlorhydrique. Aprés filtration, on cristallise deux fois dans l'isopropanol. On refroidit le produit, on le filtre et on le lave d'abord avec de l'isopropanol et après avec de l'acétone. On obtient 1,2 g (34%) du chlorhydrate de la N-[(2S)-7-hydroxy-1,2,3,4-tétrahydronapht-2-yl]-(2S)-2-(3-chlorophényl)-2-hydroxyéthanamine, SR 58575 A; p.f. 203-205°C; [alpha] = -35,8° (méthanol, c = 1%).

EXEMPLE 16

On chauffe 16 heures au reflux une solution de 2,4 g de N-[(2R)-7-hydroxy-1,2,3,4-tétrahydronapht-2-yl]-(S ou R)-mandélamide, SR 58561, obtenu comme décrit dans l'Exemple 10(b), dans 100 ml de tétrahydrofuranne, en présence de 1,85 g d'hydrure de lithium et d'aluminium. On ajoute un mélange de 5 ml d'eau dans 13 ml d'éthanol, on laisse sous agitation pendant 15 minutes et on ajoute de l'hydroxyde d'ammonium concentré. On filtre et on lave le produit ainsi obtenu avec de l'éthanol. On évapore à sec, on reprend le résidu avec de l'acétate d'éthyle. On sèche sur du sulfate de sodium anhydre et on évapore à sec. Après cristallisation du résidu dans 15 ml d'isopropanol, on obtient 1 g (44%) de N-[(2R)-7-hydroxy-1,2,3,4-tétrahydronapht-2-yl]-(2S ou 2R)-2-hydroxy-2-phényléthanamine, SR 58372; p.f. 172-174°C; [alpha] = +77,6° (DMF, c = 1%).

EXEMPLE 17

Suivant le mode opératoire décrit dans l'Exemple 16 et en utilisant comme produit de départ 2,3 g de SR 58544, obtenu comme décrit dans l'Exemple 10(c), on obtient une base résiduelle qu'on dissout dans 50 ml d'acétone et rend acide avec une solution d'éthanol saturé d'acide chlorhydrique. On cristallise le produit ainsi obtenu par dissolution dans 15 ml d'isopropanol et par précipitation avec 15 ml d'éther éthylique. On obtient 0,9 g (36%) du chlorhydrate de la N-[(2R)-7-hydroxy-1,2,3,4-tétrahydronapht-2-yl]-(2R)-2-hydroxy-2-phényléthanamine; p.f. 178-180°C; [alpha] = +40,3° (éthanol/eau 1/1, c = 2%). Ce composé correspond au produit SR 58375 A de l'Exemple 22 de EP 211721.

EXEMPLE 18

Suivant le mode opératoire décrit dans l'Exemple 16, en partant de 2,7 g de SR 58543, obtenu comme décrit dans l'Exemple 11(b), on obtient 1 g (40%) de N-[(2S)-7-hydroxy-1,2,3,4-tétrahydronapht-2-yl]-(2R ou 2S)-2-hydroxy-2-phényléthanamine, SR 58374; p.f. 173-174°C; [alpha] = -78,3° (DMF, c = 1%).

EXEMPLE 19

Suivant le mode opératoire décrit dans l'Exemple 16, en partant de 3 g de SR 58559, obtenu comme décrit dans l'Exemple 11(c), on obtient une base résiduelle qu'on dissout dans 20 ml d'acétone et on rend acide avec une solution saturée d'acide chlorhydrique dans l'isopropanol. On cristallise le produit ainsi obtenu par dissolution dans 8 ml d'isopropanol et précipitation avec 8 ml d'éther éthylique. On obtient 1,55 g (49%) du chlorhydrate de N-](2S)-7-hydroxy-1,2,3,4-tétrahydronapht-2-yl]-(2S ou 2R)-2-hydroxy-2-phényléthanamine; p.f. 179-180°C; [alpha] = -41,5° (éthanol/eau 1/1, c = 2%). Ce composé correspond au

produit SR 58373A de l'Exemple 23 de EP 211721.

EXEMPLE 20

A une suspension de 2,6 g de chlorhydrate de (R)-2-amino-7-hydrorytétraline (PREPARATION II), 2,4 g d'acide (R)-3-chloromandélique (Bull. Soc. Chim. Fr., 1973, 12, 3330) et 5,2 g de BOP dans 60 ml de chlorure de méthylène anhydre, on ajoute lentement 3,6 ml de triéthylamine, puis on laisse la solution obtenue sous agitation à la température ambiante pendant 3 heures. On ajoute 50 ml d'une solution saturée de chlorure de sodium, on laisse sous agitation pendant 30 minutes à la température ambiante, on ajoute 200 ml d'acétate d'éthyle et on sépare les phases. On lave la phase organique deux fois avec 30 ml d'une solution d'acide chlorhydrique 2N puis deux fois avec 30 ml d'une solution saturée de chlorure de sodium. On sèche la solution organique sur du sulfate de sodium, on la filtre et on l'évapore à sec. On purifie l'huile obtenue par flash chromatographie en utilisant comme solvant un mélange acétate d'éthyle/cyclohexane 55/45. On obtient un solide pâteux qu'on reprend avec 20 ml d'éther éthylique dans lequel le produit cristallise. On ajoute 10 ml de cyclohexane, on filtre, on lave avec un mélange cyclohexane/éther éthylique 2/1 et on sèche sous préssion réduite à 50 ° C. On obtient 2,4 g (55%) d'un stéréoisomère pur selon la RMN $^{13}C$ à 60 MHz; p.f. 132-134 ° C, [alpha] = + 24,9 ° (méthanol, c = 1%). Ce produit présente le même déplacement en CCM que le produit de l'Exemple 1(c), SR 58536 (RR,SS) et aucune impurêté correspondant au Rf du produit de l'Exemple 1(b), à savoir du SR 58535 (RS,SR) (éluant acétate d'éthyle/cyclohexane 1/1). Après une ultérieure purification par chromatographie, on obtient le N-[(2R)-7-hydroxy-1,2,3,4-tétrahydronapht-2-yl]-(R)-3-chloromandélamide énantiomériquement et chimiquement pur; p.f. 143-147$^{0}$C, [alpha] = +35,1 ° (méthanol, c = 1%). Ce produit est identique au composé SR 58533 de l'Exemple 8(c), dont la configuration R,R est ainsi confirmée.

EXEMPLE 21

Suivant le mode opératoire décrit dans l'Exemple 20 et en remplaçant l'acide (R)- 3-chloromandélique par l'acide (S)-3-chloromandélique (Bull. Soc. Chim. Fr., 1973, 12, 3330) on obtient le N-[(2R)-7-hydroxy-1,2,3,4-tétrahydronapht-2-yl]-(S)-3-chloromandélamide sous formè d'un solide ayant un point de fusion indéfini; [alpha] = +92,5 ° (méthanol, c = 1%). Ce produit est identique au composé SR 58588 de l'Exemple 8(b), dont la configuration (S,R) est ainsi confirmée.

EXEMPLE 22

On fait réagir sous courant d'azote 3,6 g d'acide (R)-3-chloromandélique avec 3,5 g de monohydrate de S(-)-2-amino-7-hydroxy-1,2,3,4-tétrahydronaphtalène (PREPARATION III) et 7,7 g de BOP dans 60 ml de chlorure de méthylène anhydre. On ajoute 2,7 ml de triéthylamine, on laisse la solution obtenue sous agitation 5 heures à la température ambiante, on y ajoute 400 ml d'acétate d'éthyle et on lave. On sépare la phase organique, on l'évapore à sec, on traite le résidu ainsi obtenu avec 150 ml d'hydroxyde de sodium N et on extrait par de l'éther éthylique qui est ensuite éliminé. On acidifie la phase aqueuse avec de l'acide chlorhydrique concentré, on extrait par de l'acétate d'éthyle, on lave la phase organique à l'eau, on la sèche sur du sulfate de sodium, on la filtre et l'évapore à sec. L'huile ainsi obtenue est purifiée par flash chromatographie en utilisant comme éluant un mélange acétate d'éthyle/cyclohexane 70/30. On obtient 4 g (63%) de N-[(2S)-7-hydroxy-1,2,3,4-tétrahydronapht-2-yl]-(R)-3-chloromandélamide, sous forme d'une huile blanchâtre très épaisse qui est égrenée dans l'éther de pétrole; on obtient un solide ayant un point de fusion indéfini; [alpha] = -95 ° (méthanol, c = 1%). Ce produit est identique au composé SR 58587 de l'Exemple 9(b), dont la configuration (R,S) est ainsi confirmée.

EXEMPLE 23

Suivant le mode opératoire décrit dans l'Exemple 22 et en remplaçant l'acide (R)3-chloromandélique par l'acide (S)-3-chloromandélique, on obtient le N-[(2S)-7-hydroxy-1,2,3,4-tétrahydronapht-2-yl]-(S)-3-chloromandélamide; p.f. 145-146 ° C; [alpha] = -34,2 ° (méthanol, c = 1%). Ce produit est identique au composé SR 58574 de l'Exemple 9(c), dont la configuraton (S,S) est ainsi confirmée.

EXEMPLE 24

Suivant le mode opératoire décrit dans l'Exemple 22 et en remplaçant l'acide (R)-3-chloromandélique

par une quantité équimoléculaire d'acide (R)-mandélique on obtient le N-[(2S)-7-hydroxy-1,2,3,4-tétrahydro napht-2-yl]-(R)-mandélamide; p.f. 159-160 °C; [alpha] = -113° (méthanol, c = 0,5 %). Ce produit est identique au composé SR 58543 des Exemples 3(b) et 11(b), dont la configuration (R,S) est ainsi définie.

EXEMPLE 25

Suivant le mode opératoire décrit dans l'Exemple 22 et en remplaçant l'acide (R)-3-chloromandélique par une quantité équimoléculaire d'acide (S)-mandélique on obtient le N-[(2S)-7-hydroxy-1,2,3,4-tétrahydro napht-2-yl]-(S)-mandélamide; p.f. 147-148 °C; [alpha] = -27,5° (méthanol, c = 0,5 %). Ce produit est identique au composé SR 58559 de l'Exemple 11(c), auquel la configuration (S,S) est ainsi définitivement assignée.

EXEMPLE 26

Suivant le mode opératoire décrit dans l'Exemple 22 et en remplaçant l'acide (R)-3-chloromandélique par une quantité équimoléculaire d'acide (R)-mandélique et le monohydrate de S(-)-2-amino-7-hydroxy-1,2,3,4-tétrahydronaphtalène par le monohydrate de R(+)-2-amino-7-hydroxy-1,2,3,4-tétrahydronaphtalène, on obtient le N-[(2R)-7-hydroxy-1,2,3,4-tétrahydronapht-2-yl]-(R)-mandélamide; p.f. 145-147 °C; [alpha] = +26° (méthanol, c = 1%). Ce produit est identique au composé SA 58544 des Exemples 3(c) et 10(c), auquel la configuration (R,R) est ainsi définitivement assignée.

EXEMPLE 27

Suivant le mode opératoire décrit dans l'Exemple 22 et en remplaçant l'acide (R)-3-chloromandélique par une quantité équimoléculaire d'acide (S)-mandélique et le monohydrate de S(-)-2-amino-7-hydroxy-1,2,3,4-tétrahydronaphtalène par le monohydrate de R(+)-2-anino-7-hydroxy-1,2,3,4-tétrahydronaphtalène, on obtient le N-[(2R)-7-hydroxy-1,2,3,4-tétrahydronapht-2-yl]-(S)-mandélamide; p.f. 159-160 °C; [alpha] = +110° (méthanol, c = 0,5%). Ce produit est identique au composé SR 58561 de l'Exemple 10(b), dont la configuration (S,R) est ainsi definie.

EXEMPLES 28 A 31

En soumettant les produits des Exemples 20 à 23 à une réduction avec borane-méthylsulfure comme décrit dans l'Exemple 12, on obtient les produits suivants:

- chlorhydrate de la N-[(2R)-7-hydroxy-1,2,3,4-tétrahydronapht-2-yl]-(2R)-2-(3-chlorophényl)-2-hydroxyéthanamine, identique au composé SR 58572 A de l'Exemple 13, dont la configuration (R,R) est confirmée (Exemple 28);
- N-[(2R)-7hydroxy-1,2,3,4-tétrahydronapht-2-yl]-(2S)-2-(3-chlorophényl)-2-hydroxyéthanamine, identique au composé SR 58590 de l'Exemple 12, dont la configuration (S,R) est ainsi confirmée (Exemple 29);
- N-[(2S)-7-hydroxy-1,2,3,4-tétrahydronapht-2-y-l](2R)-2-(3-chlorophényl)-2-hydroxyéthanamine, identique au composé SR 58589 de l'Exemple 14, dont la configuration (R,S) est ainsi confirmée (Exemple 30);
- chlorhydrate de la N-[(2S)-7-hydroxy-1,2,3,4-tétrahydronapht-2-yl]-(2S)-2-(3-chlorophényl)-2-hydroxyéthanamine, identique au composé SR 58575A de l'Exemple 15, dont la configuration (S,S) est ainsi confirmée (Exemple 31);

EXEMPLES 32 A 35

En soumettant les produits des Exemples 24 à 27 à une réduction avec l'hydrure de lithium et d'aluminium comme décrit dans l'Exemple 16, on obtient les produits suivants:

- N-[(2S)-7-hydroxy-1,2,3,4-tétrahydronapht-2-yl]-(2R)-2-hydroxy-2-phényléthanamine, identique au composé SR 58374 de l'Exemple 18, dont la configuration (R, S) est ainsi définie (Exemple 32) ;
- chlorhydrate de la N-[(2R)-7-hydroxy-1,2,3,4-tétrahydronapht-2-yl]-(2R)-2-hydroxy-2-phényléthanamine, identique au composé SR 58375 A de l'Exemple 17 dont la configuration (R, R) est ainsi définie (Exemple 33)
- chlorhydrate de la N-[(2S)-7-hydroxy-1,2,3,4-tétrahydronapht-2-yl]-(25)-2-hydroxy-2-phényléthanamine, identique au composé SR 58373 A de l'Exemple 19, auquel la configuration (S,S) est définitivement assignée (Exemple 34);

- N-[(2R)-7-hydroxy-1,2,3,4-tétrahydronapht-2-yl]-(2S)-2-hydroxy-2-phényléthanamine, identique au composé SR 58372 de l'Exemple 16, dont la configuration (S, R) est ainsi définie (Exemple 35).

EXEMPLE 36

On chauffe au reflux sous agitation un mélange de 15,8 g de N-[(2S)-7-hydroxy-1,2,3,4-tétrahydronapht-2-yl]-(R)-mandélamide, obtenu selon l'Exemple 3(b), 7,1 ml de bromoacétate d'éthyle, 22 g de carbonate de potassium anhydre et 1 g d'iodure de potassium dans 600 ml d'acétone anhydre. Après 5 heures et demi de chauffage, on filtre, on évapore à sec et on laisse cristalliser l'huile qui se sépare. On purifie par flash chromatographie le solide obtenu en utilisant comme éluant un mélange acétate d'éthyle/cyclohexane 1/1. On reprend et on égrène le solide de la colonne dans 20 ml d'éther éthylique, on filtre et l'on cristallise dans de l'acétate d'éthyle. On obtient 11,5 g de N-[(2S)-7-carbéthoxyméthoxy-1,2,3,4-tétrahydronapht-2-yl]-(R)-mandélamide, SR 58638; p.f. 115-117° C; [alpha] = - 98,3° (méthanol, c = 1%). Rendement: 57%.

EXEMPLE 37

A une solution de 5 g de N-[(2S)-7-carbéthoxyméthoxy-1,2,3,4-tétrahydronapht-2-yl]-(R)-mandélamide, Exemple 36, dans 50 ml de tétrahydrofuranne anhydre, on ajoute goutte à goutte pendant 10 minutes à la température de 0° C sous courant d'azote 2,6 ml d'une solution 10 M de borane-méthylsulfure dans 10 ml de tétrahydrofuranne anhydre et on garde le mélange réactionnel pendant une nuit à la température ambiante. On détruit le borane-méthylsulfure qui n'a pas réagi par addition, avec précaution, de 30 ml d'éthanol absolu et chauffage au reflux pendant 30 minutes. On évapore à sec et on opère une flash chromatographie en éluant avec un mélange acétate d'éthyle/méthanol 85/15. On sépare deux produits qu'on reprend par de l'éther éthylique. Le produit moins polaire donne, après cristallisation dans l'acétate d'éthyle, 1,2 g de N-[(2S)-7-carbéthoxyméthoxy-1,2,3,4-tétrahydronapht-2-yl]-(2R)-2-hydroxy-2-phényléthanamine, SR 58639; p.f. 108-111° C; [alpha] = -78,65° (méthanol, c = 1%). Le rendement en SR 58639 est de 25,2 %. Le rendement global à partir du N-[(2S)-7-hydroxy-1,2,3,4-tétrahydronapht-2-yl]-(R)-mandélamide est de 14,36 %.

Le produit plus polaire donne, après cristallisation dans l'acétate d'éthyle, 0,5 g de N-[(2S)-7-(2-hydroxyéthoxy-1,2,3,4-tétrahydronapht-2-yl]-(2R)-2-hydroxy-2-phényléthanamine, SR 58640; p.f. 94-96° C; [alpha] = -83,68° (méthanol, c = 1%).

EXEMPLE 38

Suivant le mode opératoire décrit dans l'Exemple 37, en utilisant les mêmes quantités des réactifs, mais chauffant au reflux pendant 3 heures, on obtient:

- 0,8 g de N-[(2S)-7-carbéthoxyméthoxy-1,2,3,4-tétrahydronapht-2-yl]-(2R)-2-hydroxy-2-phényléthanamine, SR 58639, et
- 1,8 g de N-[(2S)-7-(2-hydroxyéthoxy)-1,2,3,4-tétrahydronapht-2-yl]-(2R)-2-hydroxy-2-phényléthanamine, SR 58640, identiques aux composés de l'Exemple 37.

Le composé SR 58640 possède une bonne activité sur la motricité intestinale dans le test du côlon isolé de rat (EP 255 415).

**Revendications**

**Revendications pour les Etats contractants suivants : AT, BE, CH, DE, FR, GB, GR, IT, LI, LU, NL, SE**

**1.** Procédé pour la préparation de phényléthanolaminotétralines de formule

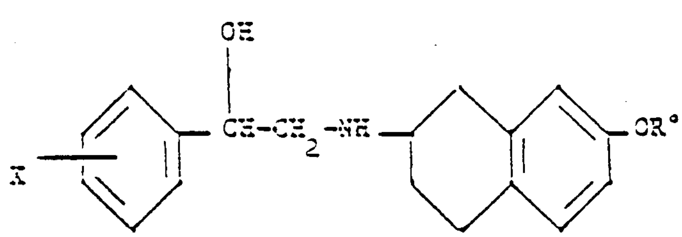

(I)

dans laquelle X représente l'hydrogène, un halogène, un groupe trifluorométhyle ou un groupe alkyle

inférieur et R° représente l'hydrogène ou un groupe méthyle substitué par un groupe carboxy ou carbalcoxy inférieur; et de leurs sels pharmaceutiquement acceptables, caractérisé en ce qu'on traite un dérivé fonctionnel d'un acide mandélique de formule

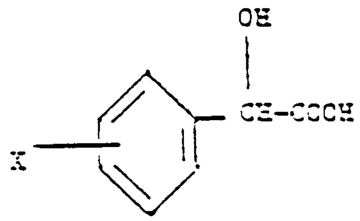

(II)

dans laquelle X est tel que défini ci-dessus, avec une aminotétraline de formule

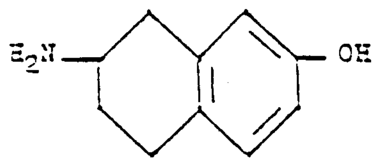

;

(III)

le mandelamide ainsi obtenu de formule

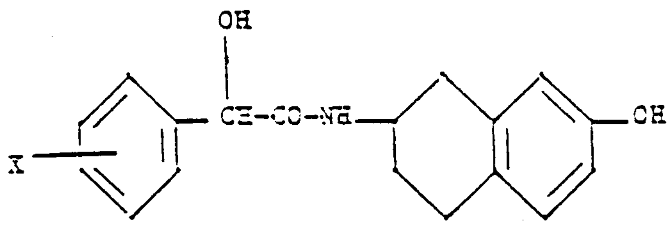

(IV)

dans laquelle X est tel que défini ci-dessus, est alors soumis à une réduction pour la transformation de son groupe amido en groupe méthylèneamino ou bien à une réaction, avec un haloacétate d'alkyle inférieur en présence d'un agent de condensation basique, ledit haloacétate étant un bromo-, chloro- ou iodoacétate ; dans cette seconde hypothèse, on soumet le produit obtenu, dans un ordre quelconque, à une réduction pour la transformation de son groupe amido en groupe méthylèneamino et à une saponification éventuelle du groupe carbalcoxy inférieur en groupe carboxy ; et on transforme éventuellement le produit ainsi obtenu en l'un de ses sels pharmaceutiquement acceptables.

**2.** Procédé selon la revendication 1, caractérisé en ce que comme produit de départ on utilise un acide mandélique dans sa configuration (R).

**3.** Procédé selon l'une quelconque des revendications 1 et 2 caractérisé en ce que comme acide mandélique de départ on utilise l'acide (R)-3-chloromandélique.

**4.** Procédé selon la revendication 1, caractérisé en ce que comme produit de départ on utilise l'acide 3-chloromandélique racémique.

**5.** Procédé selon l'une quelconque des revendications 1 à 4 caractérisé en ce que comme dérivé fonctionnel de l'acide mandélique de départ on utilise l'acide libre, activé par l'hexafluorophosphate de benzotriazolyl-N-oxytris (diméthylamino)phosphonium.

**6.** Procédé selon l'une quelconque des revendications 1 à 5 caractérisé en ce que comme aminotétraline de départ on utilise le 2-amino-7-hydroxy-1,2,3,4-tétrahydronaphtalène racémique.

**7.** Procédé selon l'une quelconque des revendications 1 à 6 caractérisé en ce que comme produit de

départ on utilise le (S)-2-amino-7-hydroxy-1,2,3,4-tétrahydronaphtalène.

**8.** Mandélamide de formule

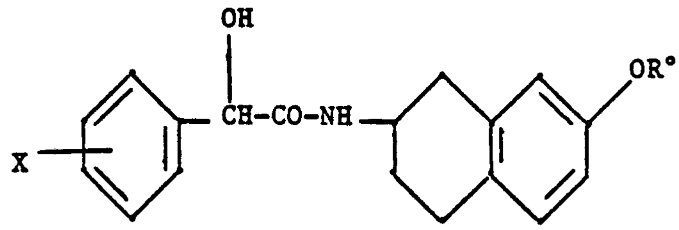

(IVa)

dans laquelle X et R° sont tels que définis dans la revendication 1.

**9.** N-(7-hydroxy-1,2,3,4-tétrahydronapht-2-yl)-3-chloromandélamide.

**10.** (RS,SR)-N-(7-hydroxy-1,2,3,4-tétrahydronapht-2-yl)-3-chloromandélamide.

**11.** (RR,SS)-N-(7-hydroxy-1,2,3,4-tétrahydronapht-2-yl)-3-chloromandélamide.

**12.** N-[(2R)-7-hydroxy-1,2,3,4-tétrahydronapht-2-yl]-(R)-3-chloromandélamide.

**13.** N-[(2S)-7-hydroxy-1,2,3,4-tétrahydronapht-2-yl]-(R)-3-chloromandélamide.

**14.** N-[(2S)-7-carbéthoxyméthoxy-1,2,3,4-tétrahydronapht-2-yl]-(R)-mandélamide.

**Revendications pour l'Etat contractant suivant : ES**

**1.** Procédé pour la préparation de phényléthanolaminotétralines de formule

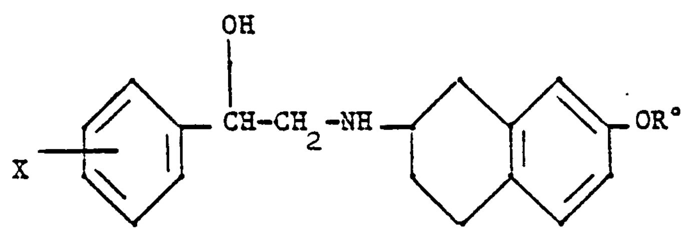

(I)

dans laquelle X représente l'hydrogène, un halogène, un groupe trifluorométhyle ou un groupe alkyle inférieur et R° représente l'hydrogène ou un groupe méthyle substitué par un groupe carboxy ou carbalcoxy inférieur; et de leurs sels pharmaceutiquement acceptables, caractérisé en ce qu'on traite un dérivé fonctionnel d'un acide mandélique de formule

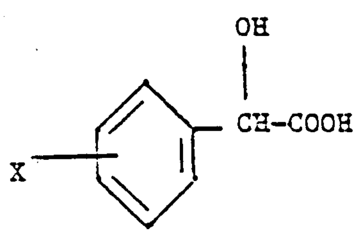

(II)

dans laquelle X est tel que défini ci-dessus, avec une aminotétraline de formule

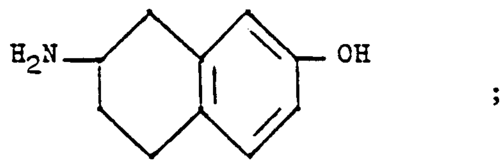

(III)

le mandélamide ainsi obtenu de formule

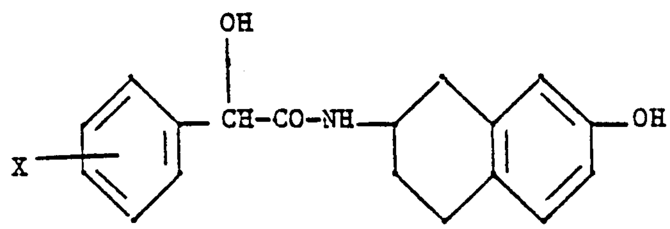

(IV)

dans laquelle X est tel que défini ci-dessus, est alors soumis à une réduction pour la transformation de son groupe amido en groupe méthylèneamino ou bien à une réaction, avec un haloacétate d'alkyle inférieur en présence d'un agent de condensation basique, ledit haloacétate étant un bromo-, chloro- ou iodoacétate ; dans cette seconde hypothèse, on soumet le produit obtenu, dans un ordre quelconque, à une réduction pour la transformation de son groupe amido en groupe méthylèneamino et à une saponification éventuelle du groupe carbalcoxy inférieur en groupe carboxy ; et on transforme éventuellement le produit ainsi obtenu en l'un de ses sels pharmaceutiquement acceptables.

2. Procédé selon la revendication 1, caractérisé en ce que comme produit de départ on utilise un acide mandélique dans sa configuration (R).

3. Procédé selon l'une quelconque des revendications 1 et 2 caractérisé en ce que comme acide mandélique de départ on utilise l'acide (R)-3-chloromandélique.

4. Procédé selon la revendication 1, caractérisé en ce que comme produit de départ on utilise l'acide 3-chloromandélique racémique.

5. Procédé selon l'une quelconque des revendications 1 à 4 caractérisé en ce que comme dérivé fonctionnel de l'acide mandélique de départ on utilise l'acide libre, activé par l'hexafluorophosphate de benzotriazolyl-N-oxytris (diméthylamino)phosphonium.

6. Procédé selon l'une quelconque des revendications 1 à 5 caractérisé en ce que comme aminotétraline de départ on utilise le 2-amino-7-hydroxy-1,2,3,4-tétrahydronaphtalène racémique.

7. Procédé selon l'une quelconque des revendications 1 à 6 caractérisé en ce que comme produit de départ on utilise le (S)-2-amino-7-hydroxy-1,2,3,4-tétrahydronaphtalène.

8. Procédé pour la préparation d'un mandélamide de formule

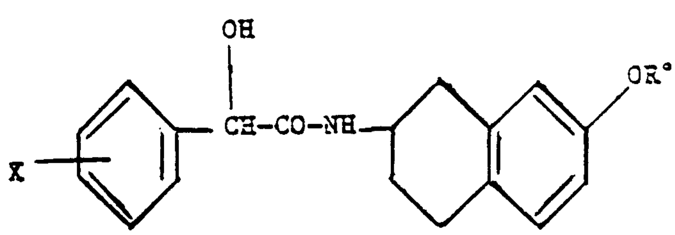

(IVa)

dans laquelle X et R° sont tels que définis dans la revendication 1, caractérisé en ce qu'on traite un dérivé fonctionnel d'un acide mandélique de formule

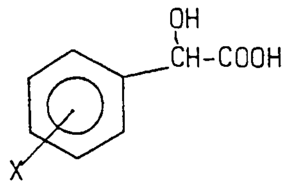

(II)

dans laquelle X est tel que défini ci-dessus, avec une aminotétraline de formule

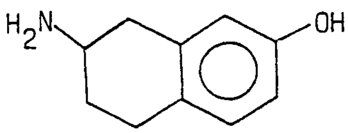

(III)

on sépare éventuellement en ses isomères le mandélamide de formule (IVa) où R° est l'hydrogène ainsi obtenu, et si on veut obtenir un mandélamide de formule (IVa) où R° est différent de l'hydrogène, on soumet le composé ainsi obtenu à une réaction avec un haloacétate d'aklyle inférieur en présence d'un agent basique, ledit haloacétate étant un bromo-, chloro- ou iodoacétate, et éventuellement à une saponification du groupe carbalcoxy inférieur en groupe carboxy.

**9.** Procédé selon la revendication 8, pour la préparation du N-(7-hydroxy-1,2,3,4-tétrahydronapht-2-yl)-3-chloromandélamide.

**10.** Procédé selon la revendications 8, pour la préparation du (RS,SR)-N-(7-hydroxy-1,2,3,4-tétrahydronapht-2-yl)-3-chloromandélamide.

**11.** Procédé selon la revendication 8, pour la préparation du (RR,SS)-N-(7-hydroxy-1,2,3,4-tétrahydronapht-2-yl)-3-chloromandélamide.

**12.** Procédé selon la revendication 8, pour la préparation du N-[(2R)-7-hydroxy-1,2,3,4-tétrahydronapht-2-yl]-(R)-chloromandélamide.

**13.** Procédé selon la revendication 8, pour la préparation du N-[(2S)-7-hydroxy-1,2,3,4-tétrahydronapht-2-yl]-(R)-3-chloromandélamide.

**14.** Procédé selon la revendication 8, pour la préparation du N-[(2S)-7-carbéthoxyméthoxy-1,2,3,4-tétrahydronapht-2-yl]-(R)-mandélamide.

**Claims**

**Claims for the following Contracting States : AT, BE, CH, DE, FR, GB, GR, IT, LI, LU, NL, SE**

**1.** Process for the preparation of phenylethanolaminotetralins of formula

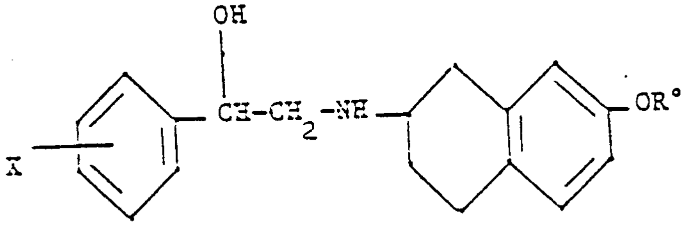

(I)

in which X is hydrogen, a halogen, a trifluoromethyl group or a lower alkyl group and R° is hydrogen or a methyl group substituted by a carboxy or a lower carbalkoxy group; and of their pharmaceutically acceptable salts, characterized in that a functional derivative of a mandelic acid of formula

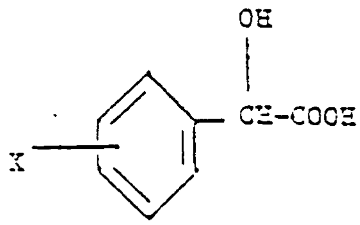

(II)

in which X is as defined hereinabove, is treated with an aminotetralin of formula

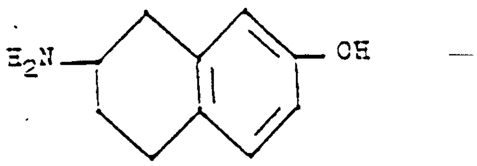

;

(III)

the thus obtained mandelamide of formula

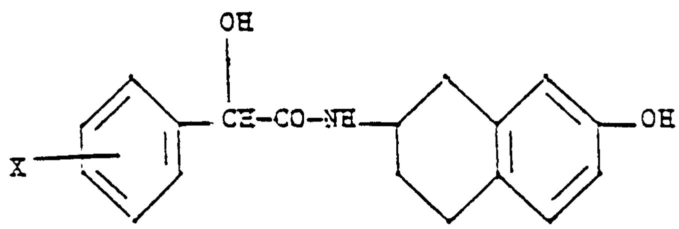

(IV)

in which X is as defined hereinabove, is then submitted to a reduction for the transformation of its amido group into a methyleneamino group or to a reaction, with a lower alkyl haloacetate in the presence of a basic condensation agent, said haloacetate being a bromo-, chloro- or iodoacetate; in this second assumption, the resulting product is submitted, in any order, to a reduction for the transformation of its amido group into a methyleneamino group and, if desired, to a saponification of the lower carbalkoxy group into the carboxy group; and, if desired, the resulting product may be transformed into one of its pharmaceutically acceptable salts.

**2.** Process according to claim 1, characterized in that a mandelic acid in its (R) configuration is used as a starting product.

**3.** Process according to any one of claims 1 and 2, characterized in that the (R)-3-chloromandelic acid is used as a starting mandelic acid.

**4.** Process according to claim 1, characterized in that the racemic 3-chloromandelic acid is used as a starting product.

**5.** Process according to any one of claims 1 to 4, characterized in that the free acid, activated by the hexafluorophosphate of benzotriazolyl-N-oxytris-(dimethylamino)phosphonium is used as a functional derivative of the starting mandelic acid.

**6.** Process according to any one of claims 1 to 5, characterized in that the racemic 2-amino-7-hydroxy-1,2,3,4-tetrahydronaphthalene is used as a starting aminotetralin.

**7.** Process according to any one of claims 1 to 6, characterized in that the (S)-2-amino-7-hydroxy-1,2,3,4-tetrahydronaphthalene is used as a starting product.

**8.** Mandelamide of formula

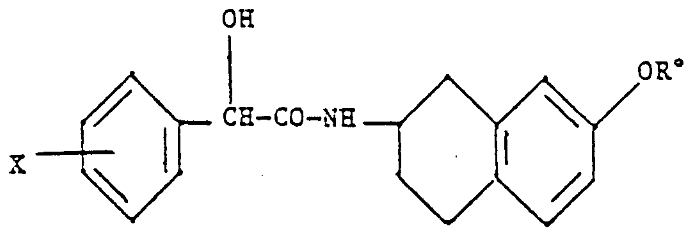

(IVa)

in which X and R° are as defined in claim 1.

**9.** N-(7-hydroxy-1,2,3,4-tetrahydronaphth-2-yl)-3-chloromandelamide.

**10.** (RS,SR)-N-(7-hydroxy-1,2,3,4-tetrahydronaphth-2-yl)-3-chloromandelamide.

**11.** (RR,SS)-N-(7-hydroxy-1,2,3,4-tetrahydronaphth-2-yl)-3-chloromandelamide.

**12.** N-[(2R)-7-hydroxy-1,2,3,4-tetrahydronaphth-2-yl]-(R)-3-chloromandelamide.

**13.** N-[(2S)-7-hydroxy-1,2,3,4-tetrahydronaphth-2-yl]-(R)3-chloromandelamide.

**14.** N-[(2S)-7-carbethoxymethoxy-1,2,3,4-tetrahydronaphth-2-yl]-(R)-mandelamide.

**Claims for the following Contracting State : ES**

**1.** Process for the preparation of phenylethanolaminotetralins of formula

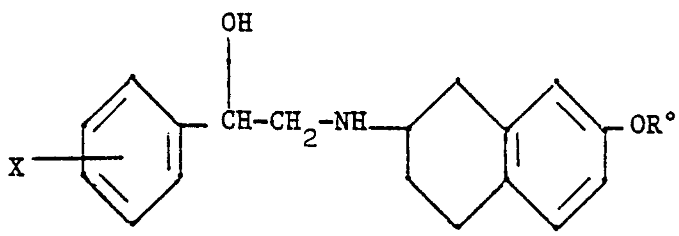

(I)

in which X is hydrogen, a halogen, a trifluoromethyl group or a lower alkyl group and R° is hydrogen or a methyl group substituted by a carboxy or a lower carbalkoxy group; and of their pharmaceutically acceptable salts, characterized in that a functional derivative of a mandelic acid of formula

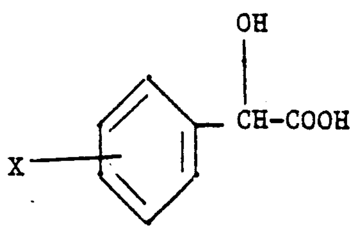

(II)

in which X is as defined hereinabove, is treated with an aminotetralin of formula

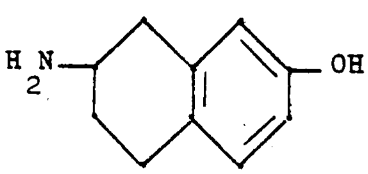

(III)

the thus obtained mandelamide of formula

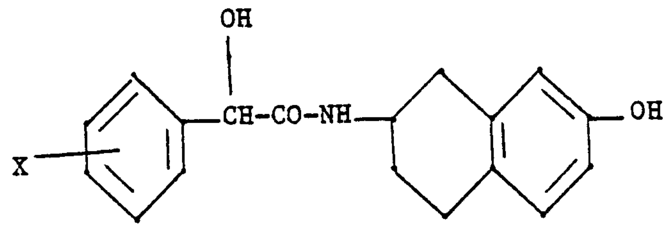

(IV)

in which X is as defined hereinabove, is then submitted to a reduction for the transformation of its amido group into a methyleneamino group or to a reaction, with a lower alkyl haloacetate in the presence of a basic condensation agent, said haloacetate being a bromo-, chloro- or iodoacetate; in this second assumption, the resulting product is submitted, in any order, to a reduction for the transformation of its amido group into a methyleneamino group and, if desired, to a saponification of the lower carbalkoxy group into the carboxy group; and, if desired, the resulting product may be transformed into one of its pharmaceutically acceptable salts.

**2.** Process according to claim 1, characterized in that a mandelic acid in its (R) configuration is used as a starting product.

**3.** Process according to any one of claims 1 and 2, characterized in that the (R)-3-chloromandelic acid is used as a starting mandelic acid.

**4.** Process according to claim 1, characterized in that the racemic 3-chloromandelic acid is used as a starting product.

**5.** Process according to any one of claims 1 to 4, characterized in that the free acid, activated by the hexafluorophosphate of benzotriazolyl-N-oxytris-(dimethylamino)phosphonium is used as a functional derivative of the starting mandelic acid.

**6.** Process according to any one of claims 1 to 5, characterized in that the racemic 2-amino-7-hydroxy-1,2,3,4-tetrahydronaphthalene is used as a starting aminotetralin.

**7.** Process according to any one of claims 1 to 6, characterized in that the (S)-2-amino-7-hydroxy-1,2,3,4-tetrahydronaphthalene is used as a starting product.

**8.** Process for the preparation of a mandelamide of formula

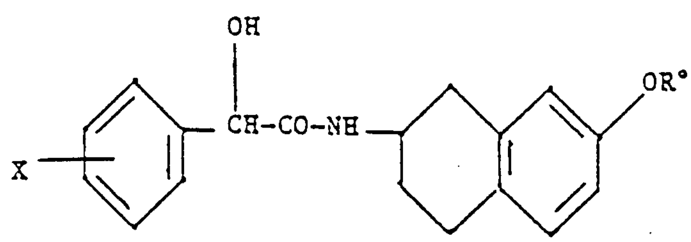

(IVa)

in which X and R° are as defined in claim 1, characterized in that a functional derivative of a mandelic acid of formula

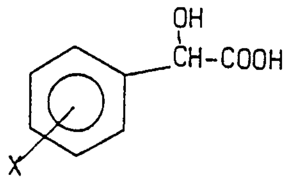

(II)

in which X is as defined hereinabove, is treated with an aminotetralin of formula

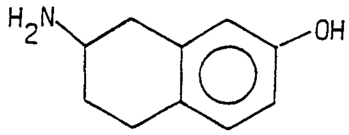

(III)

the mandelamide of formula (IVa), where R° is the thus obtained hydrogen, is optionally separated into its isomers, and, if desired, a mandelamide of formula (IVa) where R° is different from hydrogen may be obtained by submitting the thus obtained compound to a reaction with a lower alkyl haloacetate in the presence of a basic agent, said haloacetate being a bromo-, chloro- or iodoacetate and, if desired, to a saponification of the lower carbalkoxy group into a carboxy group.

**9.** Process according to claim 8, for the preparation of the N-(7-hydroxy-1,2,3,4-tetrahydronaphth-2-yl)-3-chloromandelamide.

**10.** Process according to claim 8, for the preparation of the (RS,SR)-N-(7-hydroxy-1,2,3,4-tetrahydronaphth-2-yl)-3-chloromandelamide.

**11.** Process according to claim 8, for the preparation of the (RR,SS)-N-(7-hydroxy-1,2,3,4-tetrahydronaphth-2-yl)-3-chloromandelamide.

**12.** Process according to claim 8, for the preparation of the N-[(2R)-7-hydroxy-1,2,3,4-tetrahydronaphth-2-yl]-(R)-3-chloromandelamide.

**13.** Process according to claim 8, for the preparation of the N-[(2S)-7-hydroxy-1,2,3,4-tetrahydronaphth-2-yl]-(R)-3-chloromandelamide.

**14.** Process according to claim 8, for the preparation of the N-[(2S)-7-carbethoxymethoxy-1,2,3,4-tetrahy dronaphth-2-yl]-(R)-mandelamide.

**Patentansprüche**
**Patentansprüche für folgende Vertragsstaaten : AT, BE, CH, DE, FR, GB, GR, IT, LI, LU, NL, SE**

**1.** Verfahren zur Herstellung von Phenylethanolaminotetralinen der Formel

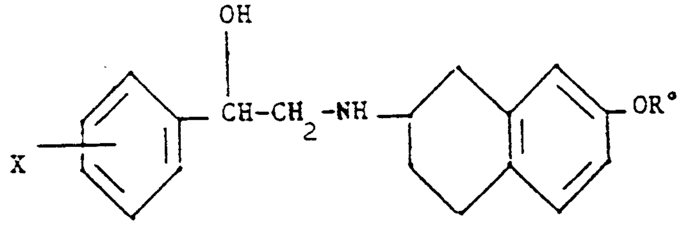

(I)

worin X Wasserstoff, ein Halogen, eine Trifluormethylgruppe oder eine nied.Alkylgruppe darstellt und R° Wasserstoff oder eine durch eine Carboxygruppe oder eine nied.Carbalkoxygruppe substituierte Methylgruppe bedeutet, und deren pharmazeutisch akzeptablen Salzen, dadurch gekennzeichnet, daß man ein funktionelles Derivat einer Mandelsäure der Formel

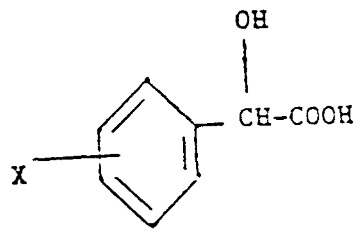

(II)

worin X wie oben definiert ist, mit einem Aminotetralin der Formel

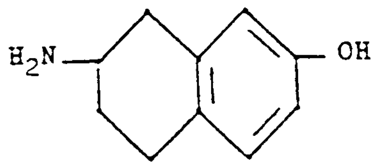

(III)

behandelt, das so erhaltene Mandelsäureamid der Formel

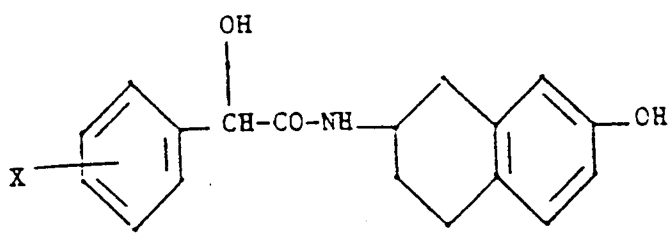

(IV)

worin X wie oben definiert ist, dann einer Reduktion zur Überführung seiner Amidogruppe in eine Methylenaminogruppe oder einer Reaktion mit einem nied.Alkylhalogenacetat in Gegenwart eines basischen Kondensationsmittels unterzieht, wobei das Halogenacetat ein Brom-, Chlor- oder Iodacetat ist, in diesem zweiten möglichen Fall das erhaltene Produkt in beliebiger Reihenfolge einer Reduktion zur Überführung seiner Amidogruppe in eine Methylenaminogruppe und gegebenenfalls einer Verseifung der nied.Carbalkoxygruppe in eine Carboxygruppe unterzieht, und gegebenenfalls das so erhaltene Produkt in eines seiner pharmazeutisch akzeptablen Salze überführt.

**2.** Verfahren nach Anspruch 1, dadurch gekennzeichnet, daß man als Ausgangsprodukt eine Mandelsäure in (R)-Konfiguration verwendet.

**3.** Verfahren nach einem der Ansprüche 1 und 2, dadurch gekennzeichnet, daß man als Ausgangsmandelsäure (R)-3-Chlormandelsäure verwendet.

**4.** Verfahren nach Anspruch 1, dadurch gekennzeichnet, daß man als Ausgangsprodukt racemische 3-Chlormandelsäure verwendet.

**5.** Verfahren nach einem der Ansprüche 1 bis 4, dadurch gekennzeichnet, daß man als funktionelles Derivat der Ausgangsmandelsäure die mit Benzotriazolyl-N-oxytris-(dimethylamino)-phosphonium-hexafluorphosphat aktivierte freie Säure verwendet.

**6.** Verfahren nach einem der Ansprüche 1 bis 5, dadurch gekennzeichnet, daß man als Ausgangsaminotetralin racemisches 2-Amino-7-hydroxy-1,2,3,4-tetrahydronaphthalin verwendet.

**7.** Verfahren nach einem der Ansprüche 1 bis 6, dadurch gekennzeichnet, daß man als Ausgangsprodukt (S)-2-Amino-7-hydroxy-1,2,3,4-tetrahydronaphthalin verwendet.

**8.** Mandelsäureamid der Formel

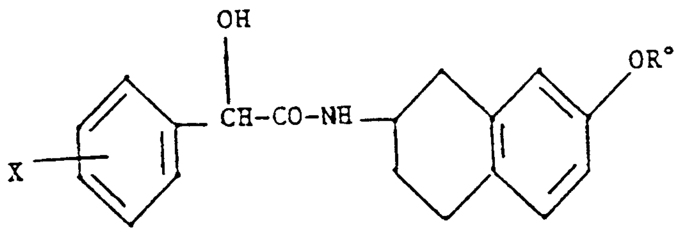

(IVa)

worin X und R° wie in Anspruch 1 definiert sind.

**9.** N-(7-Hydroxy-1,2,3,4-tetrahydronaphth-2-yl)-3-chlor-mandelsäureamid.

**10.** (RS, SR)-N-(7-Hydroxy-1,2,3,4-tetrahydronaphth-2-yl)-3 chlor-mandelsäureamid.

**11.** (RR, SS)-N-(7-Hydroxy-1,2,3,4-tetrahydronaphth-2-yl)-3 chlor-mandelsäureamid.

**12.** N-[(2R)-7-Hydroxy-1,2,3,4-tetrahydronaphth-2-Yl]-(R)-3 chlor-mandelsäureamid.

**13.** N-[(2S)-7-Hydroxy-1,2,3,4-tetrahydronaphth-2-yl]-(R)-3 chlor-mandelsäureamid.

**14.** N-[(2S)-7-Carbethoxymethoxy-1,2,3,4-tetrahydronaphth-2 yl]-(R)-mandelsäureamid.

**Patentansprüche für folgenden Vertragsstaat : ES**

**1.** Verfahren zur Herstellung von Phenylethanolaminotetralinen der Formel

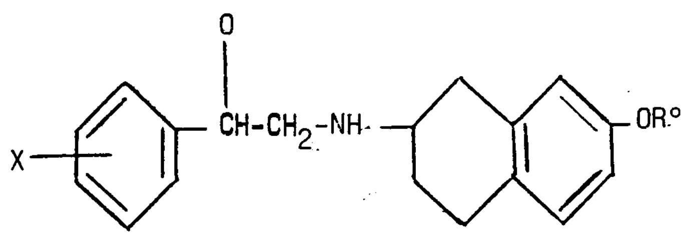

(I)

worin X Wasserstoff, ein Halogen, eine Trifluormethylgruppe oder eine nied.Alkylgruppe darstellt und R° Wasserstoff oder eine durch eine Carboxygruppe oder eine nied.Carbalcoxygruppe substituierte Methylgruppe bedeutet, und deren pharmazeutisch akzeptablen Salzen, dadurch gekennzeichnet, daß man ein funktionelles Derivat einer Mandelsäure der Formel

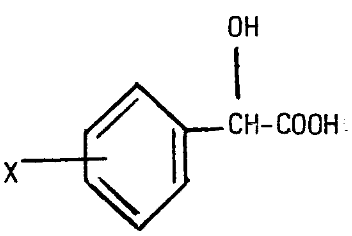

(II)

worin X wie oben definiert ist, mit einem Aminotetralin der Formel

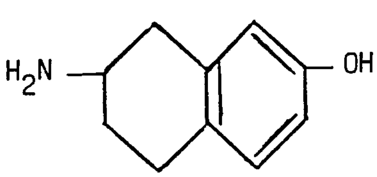

;

(III)

behandelt, das so erhaltene Mandelsäureamid der Formel

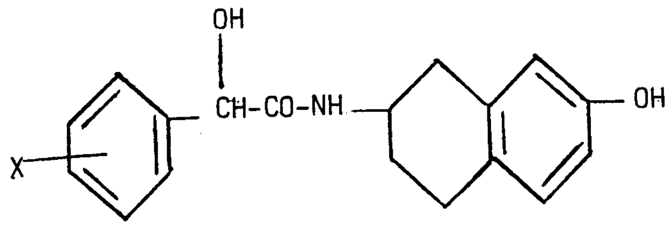

(IV)

worin X wie oben definiert ist, dann einer Reduktion zur Überführung seiner Amidogruppe in eine Methylenaminogruppe oder einer Reaktion mit einem nied.Alkylhalogenacetat in Gegenwart eines basischen Kondensationsmittels unterzieht, wobei das Halogenacetat ein Brom-, Chlor- oder Iodacetat ist, in diesem zweiten möglichen Fall das erhaltene Produkt in beliebiger Reihenfolge einer Reduktion zur Überführung seiner Amidogruppe in eine Methylenaminogruppe und gegebenenfalls einer Verseifung der nied.Carbalcoxygruppe in eine Carboxygruppe unterzieht, und gegebenenfalls das so erhaltene Produkt in eines seiner pharmazeutisch akzeptablen Salze überführt.

**2.** Verfahren nach Anspruch 1, dadurch gekennzeichnet, daß man als Ausgangsprodukt eine Mandelsäure in (R)-Konfiguration verwendet.

**3.** Verfahren nach einem der Ansprüche 1 und 2, dadurch gekennzeichnet, daß man als Ausgangsmandelsäure (R)-3-Chlormandelsäure verwendet.

**4.** Verfahren nach Anspruch 1, dadurch gekennzeichnet, daß man als Ausgangsprodukt racemische 3-Chlormandelsäure verwendet.

**5.** Verfahren nach einem der Ansprüche 1 bis 4, dadurch gekennzeichnet, daß man als funktionelles Derivat der Ausgangsmandelsäure die mit Benzotriazolyl-N-oxytris-(dimethylamino)-phosphonium-hexafluorphosphat aktivierte freie Säure verwendet.

**6.** Verfahren nach einem der Ansprüche 1 bis 5, dadurch gekennzeichnet, daß man als Ausgangsaminotetralin racemisches 2-Amino-7-hydroxy-1,2,3,4-tetrahydronaphthalin verwendet.

**7.** Verfahren nach einem der Ansprüche 1 bis 6, dadurch gekennzeichnet, daß man als Ausgangsprodukt (S)-2-Amino-7-hydroxy-1,2,3,4-tetrahydronaphthalin verwendet.

**8.** Verfahren zur Herstellung eines Mandelsäureamids der Formel

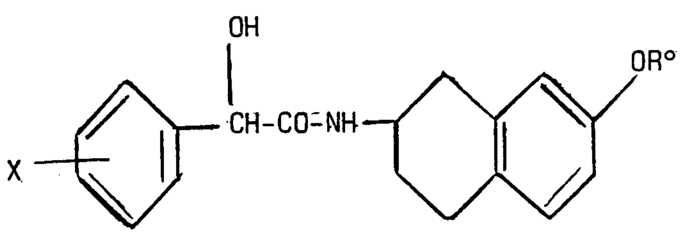

(IVa)

worin X und R° wie in Anspruch 1 definiert sind, dadurch gekennzeichnet, daß man ein funktionelles Derivat einer Mandelsäure der Formel

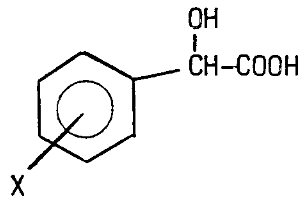

(II)

worin X wie oben definiert ist, mit einem Aminotetralin der Formel

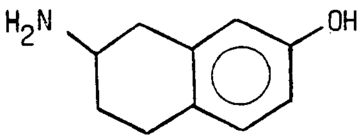

(III)

behandelt, das so erhaltene Mandelsäureamid der Formel (IVa), worin R° Wasserstoff ist, gegebenenfalls in seine Isomeren auftrennt, und, wenn ein Mandelsäureamid der Formel (IVa), worin R° nicht Wasserstoff ist, erhalten werden soll, die so erhaltene verbindung einer Reaktion mit einem nied.Alkylhalogenacetat in Gegenwart eines basischen Mittels, wobei das Halogenacetat ein Brom-, Chlor- oder Iodacetat ist, und gegebenenfalls einer Verseifung der nied.Carbalkoxygruppe in eine Carboxygruppe unterzieht.

**9.** Verfahren nach Anspruch 8 zur Herstellung des N-(7-Hydroxy-1,2,3,4-tetrahydronaphth-2-yl)-3-chlor-mandelsäureamids.

**10.** Verfahren nach Anspruch 8 zur Herstellung des (RS,SR)-N-(7-Hydroxy-1,2,3,4-tetrahydronaphth-2-yl)-3-chlor-mandelsäureamids.

**11.** Verfahren nach Anspruch 8 zur Herstellung des (RR,SS)-N-(7-Hydroxy-1,2,3,4-tetrahydronaphth-2-yl)-3-chlor-mandelsäureamids.

**12.** Verfahren nach Anspruch 8 zur Herstellung des N-[(2R)-7-Hydroxy-1,2,3,4-tetrahydronaphth-2-yl]-(R)-3-chlor-mandelsäureamids.

**13.** Verfahren nach Anspruch 8 zur Herstellung des N-[(2S)-7-Hydroxy-1,2,3,4-tetrahydronaphth-2-yl]-(R)-3-chlor-mandelsäureamids.

**14.** Verfahren nach Anspruch 8 zur Herstellung des N-[(2S)-7-Carbethoxymethoxy-1,2,3,4-tetrahydronaphth-2-yl]-(R)-mandelsäureamids.